# EUROPÄISCHE PATENTANMELDUNG

(11) **EP 4 368 696 A1**
(43) Veröffentlichungstag der Anmeldung: **15.05.2024**
(21) Anmeldenummer: 22207179.7
(22) Anmeldetag: 14.11.2022
(51) Int. Cl.: C12M 1/00

(54) **INKUBATIONSVORRICHTUNG UND MODULARES INKUBATIONSSYSTEM**

(71) Anmelder: ibidi GmbH, 82166 Gräfelfing (DE)
(72) Erfinder: Jürschick, Johannes, 80805 München (DE)
(74) Vertreter: Grünecker Patent- und Rechtsanwälte PartG mbB

(57) **Zusammenfassung**

Die Erfindung betrifft eine Inkubationsvorrichtung umfassend eine erste Inkubationskammer, wobei in der ersten Inkubationskammer ein Probenträgerhalter und eine Untersuchungsvorrichtung zur Untersuchung einer Probe, die in dem Probenträgerhalter angeordnet ist, angeordnet sind, wobei die erste Inkubationskammer eine Verbindungsöffnung zum Verbinden mit einer zweiten Inkubationskammer und eine Einlassöffnung zum Einbringen der Probe in die erste Inkubationskammer umfasst, wobei an der Verbindungsöffnung eine Verbindungseinrichtung angeordnet ist, und wobei die Verbindungseinrichtung mit einer Verbindungseinrichtung, die an einer Verbindungsöffnung einer zweiten Inkubationskammer angeordnet ist, verbindbar ist. Zudem betrifft die Erfindung ein modulares Inkubationssystem umfassend die Inkubationsvorrichtung.

## Beschreibung

Die Erfindung betrifft eine Inkubationsvorrichtung und ein modulares Inkubationssystem.

Bei der Untersuchung lebender Zellen müssen in-vivo-nahe Umgebungsbedingungen hergestellt und eingehalten werden, damit die Zellen physiologisch relevantes Verhalten zeigen und experimentelle Ergebnisse aussagekräftig und reproduzierbar sind. Dabei gibt es je nach Organ oder Zellart Unterschiede in den optimalen Bedingungen, beispielsweise betreffend die Temperatur, oder die Konzentration von Sauerstoff (O₂) oder Kohlenstoffdioxid (CO₂).

Die Zellen werden typischerweise in einem Zellmedium kultiviert, welches eine spezifische Salzkonzentration und bestimmte Nährstoffe enthält. Werden die Zellen in einem offenen System kultiviert, so verdunstet bei einer relativen Luftfeuchte unter 100 % das Zellmedium, was zu einer zeitlichen Abhängigkeit der Salzkonzentration führt. Dies sind jedoch unphysiologische Umgebungsbedingungen für die Zellen. Um Zellen stattdessen optimal kultivieren zu können, sollte um die Zellen ein abgeschlossener Raum vorhanden sein, der temperierbar, begasbar und befeuchtbar ist, damit auch über längere Zeiträume konstante und/oder kontrollierbare Bedingungen herrschen. Ein Raum, der so ansteuerbar ist, wird als Inkubationsraum oder Inkubationskammer bezeichnet. Derartige Inkubationsräume sind aus EP 2 148 921 A2 bekannt.

Wird nun den Zellen neues Zellmedium zugeführt oder sollen die Zellen unter einem ständigen Fluss stehen, wird dieses Medium zumeist von einem Reservoir mittels Schläuchen in den Bereich der Zellen geführt. Wenn dieses Medium in Schläuchen fließt, die Zonen unterschiedlicher Temperaturen durchlaufen, entstehen Temperaturunterschiede oder Schwankungen in der Konzentration des gelösten Gases im Zellmedium, wodurch sich unerwünschte Blasen bilden können, die die Flussbedingungen negativ beeinflussen. Darüber hinaus können durch Unterschiede in der relativen Luftfeuchtigkeit Kondensationsflächen entstehen, an denen in der Luft gelöstes Wasser kondensiert.

Zur Überwindung dieser Nachteile können Peripheriegeräte zur Analyse oder zum Ansteuern von inkubierten Zellträgern in externen Inkubationsräumen bereitgestellt werden. Unvorteilhaft ist dabei aber, dass dadurch zwei Inkubationssysteme angesteuert und aufeinander abgestimmt werden müssen, die trotzdem auf physiologisch relevanter Ebene unterschiedliche Umgebungsbedingungen erzeugen können und dass Schläuche in einem nicht inkubierten Bereich zwischen dem Inkubationsraum und dem Peripheriegerät verlaufen. Gerade bei kleinen Flüssen oder Flüssigkeitsmengen kann es dann innerhalb dieser Schläuche zu einem Temperaturabfall, einer Veränderung der Gaskonzentration oder zu Blasenbildung kommen. Zudem haben die Mehrzahl der unterschiedlichen Peripheriegeräte verschiedene Anforderungen und Raumbedarf, sodass sich ein komplexes System ergeben kann, das viel Raum oder Platz benötigt.

Im Lichte der genannten Nachteile ist es eine Aufgabe der vorliegenden Erfindung, eine Inkubationsvorrichtung und ein modulares Inkubationssystem bereitzustellen, die eine höhere Flexibilität ermöglicht. Diese Aufgabe wird durch eine Inkubationsvorrichtung gemäß Anspruch 1 und ein modulares Inkubationssystem gemäß Anspruch 6 gelöst.

Erfindungsgemäß wird eine Inkubationsvorrichtung bereitgestellt, umfassend:
eine erste Inkubationskammer,
wobei in der ersten Inkubationskammer angeordnet sind:
   ein Probenträgerhalter, und
   eine Untersuchungsvorrichtung zur Untersuchung einer Probe, die in dem Probenträgerhalter angeordnet ist,
wobei die erste Inkubationskammer eine Verbindungsöffnung zum Verbinden mit einer zweiten Inkubationskammer und eine Einlassöffnung zum Eingeben der Probe in die erste Inkubationskammer umfasst,
wobei an der Verbindungsöffnung eine Verbindungseinrichtung angeordnet ist, und
wobei die Verbindungseinrichtung mit einer Verbindungseinrichtung, die an einer Verbindungsöffnung der zweiten Inkubationskammer angeordnet ist, verbindbar ist.

Die erfindungsgemäße Inkubationsvorrichtung ermöglicht es, mittels der Verbindungseinrichtung weitere Inkubationsvorrichtungen oder Inkubationskammern anzubringen, um ein Inkubationssystem zu konstruieren. Ein Benutzer kann durch diesen modularen Aufbau flexibel ein Inkubationssystem gemäß seinen Anforderungen erstellen, das zum Beispiel weitere Peripheriegeräte umfassen kann. Dadurch weist die Inkubationsvorrichtung eine höhere Flexibilität auf. Typische Peripheriegeräte im Zusammenhang mit der Inkubationsvorrichtung werden nachfolgend noch genauer beschrieben.

Eine Inkubationskammer verfügt über eine Verbindungsöffnung, an der eine Verbindungseinrichtung angeordnet ist, wobei die Verbindungseinrichtung zur Verbindung einer weiteren Inkubationskammer an der Inkubationsvorrichtung, beziehungsweise der ersten Inkubationskammer dient. Hierdurch verfügt die Inkubationsvorrichtung einen modularen Charakter, sie stellt also ein Modul für ein modulares Inkubationssystem dar, das nachfolgend beschrieben wird. Weiterhin verfügt die Inkubationskammer über eine Einlassöffnung, die beispielsweise für die Eingabe einer Probe in den Probenträgerhalter oder einer Entnahme aus dem Probenträgerhalter dienen kann. Durch die Einlassöffnung können auch andere Objekte in die erste Inkubationskammer eingegeben oder aus der ersten Inkubationskammer entnommen werden oder es können Arbeiten im Inneren der ersten Inkubationskammer durchgeführt werden.

Die Inkubationsvorrichtung kann zudem eine Verschlussvorrichtung umfassen. Die Verschlussvorrichtung dient dem Verschluss der Verbindungsöffnung. Dabei kann die Verschlussvorrichtung direkt an der Verbindungsöffnung oder an der Verbindungseinrichtung angeordnet sein. Ein Verschluss mit der Verschlussvorrichtung kann insbesondere gasdicht sein.

Eine verschlossene Inkubationsvorrichtung, beziehungsweise Inkubationskammer, kann dabei als alleinstehendes Modul für die Inkubation einer Probe verwendet werden.

Eine Inkubationskammer ist ein Raum, in dem eine Temperatur, eine relative Luftfeuchtigkeit und eine Gaskonzentration, insbesondere von O₂ und CO₂ eingestellt oder insbesondere geregelt werden können. Eine solche Inkubationskammer ist besonders dazu geeignet, Zellen oder andere biologische Proben optimal zu kultivieren.

Die Untersuchungsvorrichtung kann ein Bilderfassungssystem mit einer optischen Komponente, beispielsweise ein Mikroskop mit einem Objektiv sein. Das Bilderfassungssystem kann auch die optische Komponente alleine sein.

Bei der Untersuchungsvorrichtung kann es sich auch um ein Impedanzmesssystem handeln. Dieses kann verwendet werden, um die Zelladhäsion an Oberflächen mittels Impedanzänderungen zwischen einer unterhalb der Zellen angebrachten Elektrode und einer freien Elektrode in einem Zellkulturmedium gemessen wird. Auch lassen sich in der vorliegenden Inkubationsvorrichtung mehrere Analysemethoden kombinieren.

Eine zu untersuchende Probe kann in einem in dem Probenträgerhalter gehalterten Probenträger angeordnet sein. Daher kann es synonym verstanden werden, wenn nachfolgend von einer Untersuchung einer Probe und von der Untersuchung eines Probenträgers die Rede ist.

Die Verbindungseinrichtung der ersten Kammer kann für eine formschlüssige und/oder reibschlüssige Verbindung mit einer zweiten Kammer ausgelegt sein.

Die Verbindung zwischen der ersten Inkubationskammer und der zweiten Inkubationskammer kann zerstörungsfrei lösbar sein.

Dadurch kann auch eine eventuelle Demontage der zweiten Inkubationskammer von der ersten Inkubationskammer unter geringem Aufwand erfolgen. Insbesondere handelt es sich um eine reversible Verbindung, sodass eine Montage und Demontage einer zweiten Inkubationskammer mehrfach erfolgen kann.

Die Verbindungseinrichtung kann eine Komponente einer Schraubverbindung, eines Bajonettverschlusses, einer magnetischen Verbindung mit Positioniermechanik oder einer Einhängverbindung, beispielsweise mit einem Spannverschluss, umfassen. Auch können mehrere der besagten Typen der Komponenten in der Verbindungseinrichtung kombiniert sein.

Bei den genannten Ausführungsformen der Verbindungseinrichtung handelt es sich um leicht handhabbare Verbindungsformen für mechanische Komponenten. Daher kann unter geringem Aufwand eine zweite Inkubationskammer an die Inkubationsvorrichtung angebracht werden. Auf diesem Weg wird die hohe Flexibilität der Inkubationsvorrichtung gewährleistet.

Mit anderen Worten verfügt die Kopplungseinrichtung der ersten Kammer über ein Element oder eine Komponente der oben genannten Verbindungsmechanismen. Die Verbindungseinrichtung einer zweiten Inkubationskammer, die mit der Verbindungseinrichtung der ersten Inkubationskammer verbunden wird, weist ein entsprechendes Gegenstück auf, sodass durch Verbinden der beiden Kopplungseinrichtungen eine vollständige Schraubverbindung, ein Bajonettverschluss oder eine Einhängverbindung gebildet wird.

Außerdem können mehrere Arten oder Typen zur Verbindung der Verbindungseinrichtung vorliegen. So kann beispielsweise eine Einhängverbindung zusätzlich mit einer Schraubverbindung gesichert werden. Hierdurch kann eine stabilere Verbindung zwischen der ersten Inkubationskammer und der zweiten Inkubationskammer hergestellt werden.

Die Verbindungsöffnung kann eine Fläche von höchstens 1 m², insbesondere von höchstens 0,25 m² aufweisen.

Grundsätzlich kann die Fläche der Verbindungsöffnung gemäß der vorgesehenen Verwendung der Inkubationsvorrichtung gewählt werden. Dabei soll jedoch sichergestellt werden, dass ein Gasaustausch zwischen der ersten Inkubationskammer und einer verbundenen zweiten Inkubationskammer stattfindet, sodass in beiden Inkubationskammern die gleichen Bedingungen herrschen. Dieser Austausch findet ohne zusätzliche Einrichtungen mittels Diffusion statt, wobei die Diffusionsrate von der Fläche der Verbindungsöffnung abhängt. Zudem muss die Verbindungsöffnung eine größere Fläche besitzen als eventuell vorhandene Öffnungen zu einer Umgebung der Inkubationsvorrichtung, da andernfalls keine stabilen und isotropen Umgebungsbedingungen im Inneren gewährleistet werden können. Öffnungen zur Umgebung können vorhanden sein, um beispielsweise kontrolliert Luft aus der Umgebung in die Inkubationskammer zu saugen.

Die Inkubationsvorrichtung kann weiterhin eine Schnittstelle in einer Außenwand der ersten Inkubationskammer umfassen, wobei die Schnittstelle über einen Anschluss für eine elektrische, gasführende und/oder flüssigkeitsführende Verbindung verfügt.

Die Schnittstelle kann mit einer entsprechenden Schnittstelle einer zweiten Inkubationskammer verbunden werden, sodass eine geschlossene Leitung für Gase und/oder Flüssigkeiten (Fluide) zwischen der ersten Inkubationskammer und der zweiten Inkubationskammer entsteht. Die Fluide können beispielsweise zur Versorgung einer in dem Probenträgerhalter angeordneten Probe dienen. Durch die Ausbildung einer Schnittstelle können die Leitungen in der ersten Inkubationskammer automatsch bei Montage der zweiten Inkubationskammer verbunden werden. Dadurch ist es beispielsweise auch nicht nötig, die Leitung manuell durch die Verbindungsöffnungen der beiden Kammern zu verlegen. Dies vereinfacht den Aufbau eines Inkubationssystems und ermöglicht eine kompakte Bauweise.

Elektrische Verbindungen können beispielsweise in Form von druckelastischen Pogo-Pins oder Federklemmkontakten ausgebildet sein. Über die elektrische Verbindung in der Schnittstelle kann eine in der Inkubationsvorrichtung angeordnete elektrische Einrichtung, beispielsweise Pumpen, elektrisch betriebene Ventile oder Heizelemente, mit einer Strom- und Spannungsversorgung verbunden werden, die außen an der Schnittstelle angeschlossen wird. Gleichsam kann eine zweite Inkubationskammer an die Inkubationsvorrichtung angeschlossen werden, wobei die elektrische Verbindung der Schnittstelle der Inkubationsvorrichtung und die elektrische Verbindung der Schnittstelle der zweiten Inkubationskammer verbunden werden. In beiden Fällen tragen elektrische Verbindungen in der Schnittstelle der Inkubationsvorrichtung zu einer höheren Flexibilität der beschriebenen Inkubationsvorrichtung bei.

Die Schnittstelle kann insbesondere einen Anschluss zur Übertragung von Steuerungs- oder Regelsignalen umfassen. Typische Anschlüsse in diesem Zusammenhang sind CAN-Bus, serielle Kommunikation, I2C und Ethernet. Im Fall einer nachfolgend beschriebenen Inkubationsvorrichtung oder eines nachfolgend beschriebenen Inkubationssystems mit einer Regeleinrichtung werden Regelungssignale genutzt, um aktive Komponenten der Inkubationsvorrichtung oder des Inkubationssystems, beispielsweise Pumpen oder Heizelemente, auf Basis eines entsprechenden Messwerts zu regeln. Der in der Schnittstelle vorhandene Anschlüsse Anschluss zur Übertragung von Steuerungs- oder Regelsignalen erleichtern die Verbindung der zusammengehörigen Komponenten eines Regelkreises, ermöglichen einen modularen Aufbau und erhöhen dadurch die Flexibilität der Inkubationsvorrichtung oder eines Inkubationssystems.

Die Schnittstelle kann eine Steckverbindung, insbesondere mit einem Dichtungselement, umfassen.

Steckverbindungen zeichnen sich durch eine einfache Handhabung und eine Verwendung über eine Vielzahl von Zyklen aus. Daher kann die Schnittstelle der ersten Inkubationskammer leicht und reversibel mit einer zugehörigen Schnittstelle der zweiten Inkubationskammer verbunden und die Verbindung wieder gelöst werden.

Diese Steckverbindung kann beispielsweise in Form einer Luer- oder Luerlock-Verbindung ausgebildet sein. Dabei umfasst die Schnittstelle der ersten Inkubationskammer einen weiblichen Luer- oder Luerlock Adapter, in den ein entsprechendes Gegenstück in Form eines männlichen Luer oder Luerlock-Adapters, der an einer Schnittstelle einer zweiten Inkubationskammer ausgebildet ist, eingesteckt werden kann. Durch Verwendung eines Dichtelements kann eine abgedichtete Verbindung hergestellt werden. Die Steckverbindung, insbesondere eine Luer- oder Luerlock-Verbindung kann im Zusammenhang mit Flüssigkeiten und/oder Gasen verwendet werden und stellt einen einfach handhabbaren und verlässlichen Mechanismus dar.

Insbesondere kann zwischen der Schnittstelle der ersten Inkubationskammer und dem Probenträgerhalter oder der Probe eine Verbindung für Gase und/oder Flüssigkeiten vorhanden sein, um ein Gas oder eine Flüssigkeit, das/die an der Schnittstelle in die erste Inkubationskammer geleitet wird, an die Probe, die im Probenträgerhalter angeordnet ist, zu transportieren. Die Verbindung kann zum Beispiel mittels eines Schlauchs hergestellt werden.

Die erste Inkubationskammer kann eine zweite Verbindungsöffnung umfassen, an der eine zweite Verbindungseinrichtung angeordnet ist.

Durch dieses Merkmal wird die Flexibilität der Inkubationsvorrichtung zusätzlich erhöht, weil neben einer zweiten Inkubationskammer noch eine weitere Inkubationskammer angebracht werden kann. Ein auf diese Weise konstruiertes Inkubationssystem kann beispielsweise zusätzliche Peripheriegeräte umfassen und somit weitere Einsatzmöglichkeiten bereitstellen.

Die zuvor gemachten Ausführungen betreffend die Verbindungsöffnung und die daran angeordnete Verbindungseinrichtung können in der gleichen Weise auch auf die zweite Verbindungsöffnung und die zweite Verbindungseinrichtung zutreffen. Allerdings muss dies nicht der Fall sein und die erste und zweite Verbindungsöffnung, sowie die erste und zweite Verbindungseinrichtung können unterschiedlich ausgebildet sein.

Die Inkubationsvorrichtung kann zudem über eine zweite Schnittstelle verfügen. Insbesondere kann die Anzahl der Schnittstellen der Anzahl der Verbindungsöffnungen entsprechen.

Wie beschrieben dienen die Verbindungsöffnungen dem Anschluss einer weiteren Inkubationskammer an die Inkubationsvorrichtung. Damit einhergehend kann es von Vorteil sein, für die Verbindung der zweiten Kammer auch eine zugehörige Schnittstelle bereitzustellen. Dies muss aber nicht zwingend vorliegen.

Die Inkubationsvorrichtung kann weiterhin eine Proben-Versorgungseinrichtung zur Bereitstellung eines Gases oder einer Flüssigkeit für die Probe umfassen, wobei die Proben-Versorgungseinrichtung insbesondere eine Pumpe mit einem Flüssigkeitsreservoir umfasst. Die Proben-Versorgungseinrichtung kann insbesondere im Inneren der Inkubationsvorrichtung angeordnet sein.

Eine Proben-Versorgungseinrichtung bezeichnet ein Gerät, das konfiguriert ist, ein fluides, insbesondere flüssiges, Nährmedium mit genau definierten Eigenschaften für eine biologische Probe bereitzustellen. Zu besagten Eigenschaften zählen unter anderem die Temperatur und eine Gaskonzentration, insbesondere die Konzentration von O₂ und CO₂, in dem Nährmedium. Darüber hinaus ist eine Proben-Versorgungseinrichtung in der Lage, das Nährmedium an eine Probe in der ersten Inkubationskammer zu transportieren.

Zudem stellt eine Pumpe mit einem Flüssigkeitsreservoir eine einfache Möglichkeit dar, eine Probe in dem Probenträgerhalter mit besagter Flüssigkeit oder besagtem Nährmedium zu versorgen, indem das Nährmedium durch ein Verbindungselement, beispielsweise einen Schlauch, zu der Probe gepumpt wird.

Die Inkubationsvorrichtung und insbesondere die Verbindungseinrichtung können thermisch isoliert sein.

Eine Inkubationsvorrichtung oder ein Inkubationssystem zeichnet sich dadurch aus, dass im innerhalb der Inkubationskammern stabile und kontrollierte Bedingungen, insbesondere eine konstante Temperatur, herrschen. Eine thermische Isolation sorgt dafür, dass ein Wärmeaustausch zwischen dem Inneren der Inkubationskammer und der Umgebung unterdrückt wird und die Temperatur im Inneren der Inkubationskammer besonders stabil ist. Die Verbindungseinrichtungen besonders anfällig für einen solchen Wärmeaustausch sein. Daher trägt eine thermische Isolierung der Verbindungseinrichtung zusätzlich zum beschriebenen Effekt bei.

Die Inkubationsvorrichtung kann weiterhin ein Heizelement zur Einstellung einer Temperatur in der ersten Inkubationskammer umfassen. Ferner kann die Inkubationsvorrichtung eine Kammer-Versorgungseinrichtung zur Einstellung einer Luftfeuchtigkeit und/oder Gaskonzentration, insbesondere O₂- oder CO₂-Konzentration in der ersten Inkubationskammer umfassen. Dabei können das Heizelement und/oder die Kammer-Versorgungseinrichtung insbesondere innerhalb der ersten Inkubationskammer angeordnet sein.

Die Inkubationsvorrichtung kann weiterhin einen Luftverteiler, beispielsweise einen Ventilator, umfassen.

Durch den Luftverteiler wird ein Gradient in einer Temperatur, Gaskonzentration oder der Luftfeuchtigkeit innerhalb der Inkubationskammer beschleunigt abgebaut, sodass schnell gleiche Bedingungen im Inneren Inkubationskammern hergestellt werden können.

Das Inkubationssystem kann weiterhin einen Temperatursensor, Luftfeuchtesensor und/oder Gaskonzentrationssensor zur Bestimmung mindestens eines Parameters von Temperatur, Luftfeuchtigkeit und Gaskonzentration in der ersten Inkubationskammer umfassen.

Dabei können auch mehrere Sensoren des gleichen Typs, also Sensoren die den gleichen Parameter bestimmen, umfasst sein. Auch kann ein Sensor, der alle genannten Parameter bestimmen kann, umfasst sein.

Die genannten Sensoren dienen der Überwachung und Kontrolle der Bedingungen im Inneren der Inkubationskammer beziehungsweise der Inkubationsvorrichtung. Beispielsweise können die bestimmten oder gemessenen Parameter einem Benutzer der Inkubationsvorrichtung angezeigt werden. Im Fall einer Abweichung eines Parameters kann der Benutzer gegebenenfalls Änderungen an den Einstellungen, insbesondere der Proben-Versorgungseinrichtung, der Kammer-Versorgungseinrichtung oder dem Heizelement, vornehmen, um die Parameter wieder an einen vorgesehenen Wert anzupassen.

Die Inkubationsvorrichtung kann weiterhin eine Regeleinrichtung, die ausgebildet ist, den besagten Parameter auf Grundlage eines zugehörigen Messwerts von dem Sensor oder den Sensoren zu regeln, umfassen.

Hierbei impliziert der Begriff "regeln", dass es sich um eine feedbackgesteuerte Regeleinrichtung handelt. Dabei werden die oben genannten Parameter, oder zumindest einer davon, von der Regeleinrichtung kontinuierlich an einen vorgegebenen Sollwert angepasst. Hierzu empfängt die Regeleinrichtung Messwerte von den Sensoren in Form eines elektronischen Signals, aus dem der zugehörige Parameterwert bestimmt wird. Die Regeleinrichtung umfasst zudem eine Kontrolleinrichtung, mit der die Funktion der Kammer-Versorgungseinrichtung, des Heizelements und der Proben-Versorgungseinrichtung gesteuert werden kann. Auf diese Weise nimmt die Regeleinrichtung Einfluss auf die Bedingungen im Inneren der Inkubationsvorrichtung und kann die Parameter entsprechend steuern.

Um eine Feedbackschleife zu realisieren, kann der Regeleinrichtung zudem ein Sollwert für einen bestimmten Parameter vorgegeben sein. Dieser Sollwert kann zum Beispiel von einem Benutzer eingegeben werden. Auf Basis dieses Sollwerts und des Sensorsignals errechnet die Regeleinrichtung ein Fehlersignal, aus dem hervorgeht, wie der betreffende Parameter geändert werden muss. Entsprechend greift die Regeleinrichtung auf die Versorgungseinrichtungen der Inkubationsvorrichtung zu, um die Messwerte an den Sollwert anzugleichen. Diese Operation kann simultan für mehrere der oben genannten Parameter durchgeführt werden. Zudem wird die Regelung insbesondere kontinuierlich vorgenommen.

Zu den Vorteilen einer derartigen Regelungseinrichtung zählt, dass sie eine kontinuierliche Regelung der Umgebungsbedingungen im Inneren der Inkubationsvorrichtung vornimmt, ohne dass ein Benutzer aktiv eingreifen muss. Außerdem ist die Genauigkeit der Einstellung eines bestimmten Parameters durch eine Regeleinrichtung höher als bei einem menschlichen Benutzer, was bei der Kultivierung einer biologischen Probe von hoher Wichtigkeit ist. Anders gesagt erlaubt eine Regeleinrichtung stabile Bedingungen über lange Zeiträume und eine präzise Einstellung der Parameter an einen vorgegebenen Sollwert.

Die Erfindung stellt auch ein modulares Inkubationssystem bereit, umfassend:
eine vorstehend beschriebene Inkubationsvorrichtung, und
eine zweite Inkubationskammer mit einer Verbindungsöffnung,
wobei an der Verbindungsöffnung der zweiten Inkubationskammer eine Verbindungseinrichtung angeordnet ist,
wobei die Verbindungseinrichtung der ersten Inkubationskammer mit der Verbindungseinrichtung der zweiten Inkubationskammer verbunden ist.

Das Inkubationssystem erfüllt die Verteile hinsichtlich einer erhöhten Flexibilität wie sie bereits im Hinblick auf die Inkubationsvorrichtung diskutiert wurden. Dies wird unter anderem durch den modularen Aufbau des Inkubationssystems erreicht.

Die erste Inkubationskammer und die zweite Inkubationskammer können gasdicht verbunden sein.

Dadurch ist das Inkubationssystem besser gegenüber der Umgebung isoliert, weil kein Gasaustausch mit der Umgebung stattfindet und die im Zusammenhang mit der thermischen Isolierung der Inkubationsvorrichtung genannten Vorteile kommen zum Tragen.

Das Inkubationssystem kann weiterhin eine Proben-Versorgungseinrichtung zur Bereitstellung eines Gases oder einer Flüssigkeit für die in dem Probenträgerhalter angeordnete Probe umfassen. Die Proben-Versorgungseinrichtung kann insbesondere eine Pumpe mit einem Flüssigkeitsreservoir umfassen.

Die Proben-Versorgungseinrichtung kann in der zweiten Inkubationskammer angeordnet und über ein Verbindungselement, insbesondere einen Schlauch, mit dem Probenträgerhalter oder der Probe verbunden sein.

Durch die Anordnung der Proben-Versorgungseinrichtung in der zweiten Kammer wird die Modularität des Inkubationssystems unterstrichen. Sobald für das Inkubationssystem eine Proben-Versorgungseinrichtung benötigt wird, kann eine entsprechende zweite Inkubationskammer mit einer darin angeordneten Proben-Versorgungseinrichtung an die Inkubationsvorrichtung angebracht werden. Das Inkubationssystem kann also einem individuellen Bedarf angepasst werden. Außerdem ist es möglich, ein kompaktes Inkubationssystem zu konstruieren, weil nicht alle Komponenten stets Teil des Inkubationssystems oder der Inkubationsvorrichtung sein müssen.

Die Proben-Versorgungseinrichtung kann mit dem Probenträgerhalter oder dem Probenträger verbunden sein. Diese Verbindung kann direkt ausgebildet sein. Als Verbindung kann zum Beispiel ein Schlauch verwendet werden. Im Fall einer direkten Verbindung kann diese durch die Verbindungsöffnungen der ersten und zweiten Inkubationskammer hindurch erfolgen. Beispielsweise wird der Schlauch durch die beiden Verbindungsöffnungen verlegt.

In einer Außenwand der zweiten Inkubationskammer kann eine Schnittstelle für einen Anschluss einer gasführendenden und/oder flüssigkeitsführenden Verbindung angeordnet sein, wobei die Schnittstelle der ersten Inkubationskammer mit der Schnittstelle der zweiten Inkubationskammer verbunden ist, sodass eine geschlossene gasführende und/oder flüssigkeitsführende Verbindung vorliegt.

Eine weitere mögliche Variante ist, dass die Außenwand der ersten Inkubationskammer und/oder der zweiten Inkubationskammer abnehmbar ist oder abnehmbare Teile umfasst. Beispielsweise kann die erste Inkubationskammer quaderförmig sein und eine oder mehrere der Seitenwände oder Teile der Seitenwände können abnehmbar sein. Dies dient dem Zweck, eine weitere Inkubationskammer anstelle des abnehmbaren Teils der Außenwand an der Inkubationsvorrichtung anzubringen. Für die Anbringung der zweiten Inkubationskammer kann dabei die gleiche Verbindungseinrichtung verwendet werden wie für die Anbringung der Seitenwand. Der abnehmbare Teil der Außenwand kann derart ausgebildet sein, dass er im montierten Zustand die Schnittstelle in der Außenwand der Inkubationskammer verschließt (im Fall einer gasführenden oder flüssigkeitsführenden Verbindung) oder verdeckt (im Fall einer elektrischen Verbindung). Sobald der abnehmbare Teil entfernt wird, wird die Schnittstelle geöffnet beziehungsweise freigelegt und kann mit der zugehörigen Schnittstelle einer weiteren Inkubationskammer verbunden werden.

Im Fall einer Verbindung über die Schnittstelle der ersten Inkubationskammer und der zweiten Inkubationskammer besteht jeweils eine Verbindung zwischen der Proben-Versorgungseinrichtung und der Schnittstelle der zweiten Inkubationskammer, sowie zwischen der Schnittstelle der ersten Inkubationskammer und dem Probenträgerhalter oder dem Probenträger. In dem Fall, dass die Proben-Versorgungseinrichtung in der zweiten Kammer angeordnet ist, kann eine Verbindung, beispielweise in Form eines Schlauchs von der Proben-Versorgungseinrichtung an die Schnittstelle der zweiten Kammer führen. Dort besteht eine Verbindung mit der Schnittstelle der ersten Kammer, die insbesondere gasdicht und flüssigkeitsdicht ist. Von der Schnittstelle der ersten Inkubationskammer führt eine weitere Verbindung an den Probenträgerhalter oder den Probenträger.

Das von der Proben-Versorgungseinrichtung bereitgestellte Nährmedium soll an einer Probe bereitgestellt werden, die in einem Probenträger angeordnet ist. Dieser Probenträger kann in dem Probenträgerhalter gehaltert sein. Dazu kann der Probenträgerhalter entsprechende Anschlüsse oder Ventile verfügen. Wenn der Probenträger in den Probenträgerhalter eingesetzt wird, öffnet sich ein Flüssigkeitsweg vom Probenträgerhalter in den Probenträger, sodass das Nährmedium an die Probe gelangt. Alternativ kann es nötig sein, dass das Verbindungselement von der Proben-Versorgungseinrichtung oder der Schnittstelle direkt an den Probenträger angeschlossen wird, sobald dieser in dem Probenträgerhalter gehaltert ist.

Da die erste Inkubationskammer und die zweite Inkubationskammer einen gemeinsamen inkubierten Raum bilden, herrschen in beiden Inkubationskammern dieselben Umgebungsbedingungen. Insbesondere treten dadurch keine Temperaturgradienten innerhalb des Systems auf. Im Fall einer Flüssigkeit mit einer bestimmten Gaskonzentration kann es bei einem Transport durch einen Temperaturgradienten (eine Kältebrücke) zu unerwünschter Blasenbildung kommen, wenn das gelöste Gas ausgast. Dabei kann Blasenbildung die Flussbedingungen negativ beeinflussen. Dieser Nachteil wird durch das beschriebene Inkubationssystem behoben, weil das Nährmedium durch eine Umgebung konstanter Temperatur geleitet wird.

Das Inkubationssystem kann weiterhin ein Heizelement umfassen, das ausgebildet ist, eine Temperatur in der ersten Inkubationskammer und/oder der zweiten Inkubationskammer einzustellen. Das Inkubationssystem kann ferner eine Kammer-Versorgungseinrichtung umfassen, die ausgebildet ist, eine Temperatur, Luftfeuchtigkeit und/oder Gaskonzentration, insbesondere CO₂- und/oder O₂-Konzentration, in der ersten Inkubationskammer und/oder der zweiten Inkubationskammer einzustellen.

Das Heizelement und/oder die Kammer-Versorgungseinrichtung können insbesondere innerhalb der ersten Inkubationskammer und/oder innerhalb der zweiten Inkubationskammer angeordnet sein.

In herkömmlichen Inkubationssystemen werden in der Regel externe Inkubationskammer-Versorgungseinrichtungen bereitgestellt, die die Temperatur, Luftfeuchtigkeit und Gaskonzentration im Inneren des Systems steuern. Durch Bereitstellen eines Heizelements und/oder einer Kammer-Versorgungseinrichtung innerhalb des Inkubationssystems ergeben sich zwei Vorteile. Erstens kann das System kompakter ausgestaltet werden, weil kein zusätzliches externes Gerät vorhanden ist, sondern das Heizelement und/oder die Kammer-Versorgungseinrichtung bereits in das System integriert sind. Zweitens werden die von dem Heizelement und/oder der Kammer-Versorgungseinrichtung bereitgestellten Umgebungsparameter unmittelbar an das Innere des Inkubationssystems übergeben. Ein externes Gerät müsste bereitgestellte Gase oder Flüssigkeiten zunächst über eine Verbindung in das Innere der Inkubationskammer leiten. Durch imperfekte Isolierung dieser Verbindung kann es beispielsweise zu einer Temperaturänderung infolge einer Kältebrücke kommen, sodass die Einstellungen an der externen Inkubationskammer-Versorgungseinrichtung nicht den finalen Bedingungen im Inneren des Systems entsprechen. Dieser Nachteil tritt für die hier beschriebene Konfiguration nicht auf.

Das Heizelement kann ausgebildet sein, die Temperatur in der ersten Inkubationskammer und in der zweiten Inkubationskammer unabhängig voneinander einzustellen. Ferner kann die Kammer-Versorgungseinrichtung ausgebildet sein, die Luftfeuchtigkeit und/oder die Gaskonzentration in der ersten Inkubationskammer und in der zweiten Inkubationskammer unabhängig voneinander einzustellen.

Einerseits kann es vor Vorteil sein, wenn in beiden Inkubationskammern nicht die gleichen Bedingungen herrschen. Beispielsweise können mit der Inkubationsvorrichtung Langzeitexperimente durchgeführt werden, bei der ein Nährmedium ein der zweiten Inkubationskammer gelagert wird, bevor es an die Probe in der ersten Inkubationskammer geleitet wird. In diesem Fall sollte das Nährmedium bei niedrigen Temperaturen gelagert werden, um dessen Haltbarkeit zu verlängern. Eine niedrige Temperatur bedeutet insbesondere eine kältere Umgebung als bei einer Kultivierung von Zellen. Umgekehrt kann auch die zweite Inkubationskammer auf eine höhere Temperatur eingestellt werden als die erste Inkubationskammer. Dies hat als Vorteil, dass eine Blasenbildung im Nährmedium in der kühleren ersten Inkubationskammer zusätzlich reduziert wird.

Andererseits können die beiden Inkubationskammern auch auf die gleichen Parameter eingestellt werden. Hierbei werden die beiden Inkubationskammern jeweils angesteuert ohne dass eine Angleichung durch Diffusion stattfinden muss. Entsprechend wird ein Gleichgewicht der Bedingungen im Inneren des Inkubationssystems schneller erreicht.

Das Inkubationssystem kann weiterhin einen Luftverteiler, beispielsweise einen Ventilator, für einen Austausch einer Temperatur, Gaskonzentration und/oder Luftfeuchtigkeit zwischen der ersten Inkubationskammer und der zweiten Inkubationskammer umfassen.

Durch den Luftverteiler wird ein Gradient in einer Temperatur, Gaskonzentration oder der Luftfeuchtigkeit zwischen den beiden Inkubationskammern beschleunigt abgebaut, sodass schnell gleiche Bedingungen in beiden Inkubationskammern hergestellt werden können. Beispielsweise kann dies relevant sein, wenn der Austausch, der durch Diffusion durch die Versorgungsöffnung hindurch stattfindet, aufgrund der Größe der Versorgungsöffnung begrenzt ist.

Die erste Inkubationskammer und/oder die zweite Inkubationskammer können thermisch gegenüber der Umgebung isoliert sein. Insbesondere können die Verbindungseinrichtungen der ersten Inkubationskammer und der zweiten Inkubationskammer gegenüber der Umgebung thermisch isoliert sein.

Das Inkubationssystem kann weiterhin einen Temperatursensor, einen Luftfeuchtigkeitssensor oder einen Gaskonzentrationssensor zur Bestimmung eines Parameters von Temperatur, Luftfeuchtigkeit und/oder Gaskonzentration in der ersten Inkubationskammer und/oder der zweiten Inkubationskammer umfassen.

Betreffend die Art der Sensoren treffen die gleichen Eigenschaften zu wie sie im Zusammenhang mit der Inkubationsvorrichtung diskutiert wurden.

Die Sensoren können dabei an unterschiedlichen Stellen innerhalb des Inkubationssystems angebracht sein. Beispielsweise können ein oder mehrere Sensoren am Probenträgerhalter oder in dessen unmittelbarer Umgebung angebracht sein, um die Bedingungen in einer in dem Probenträgerhalter angeordneten Probe zu überwachen. Gleichsam können auch Sensoren in beiden Inkubationskammern vorhanden sein, um beispielsweise sicherzustellen, dass innerhalb des Inkubationssystems konstante und isotrope Bedingungen herrschen. Die Anordnung der Sensoren kann auf alle genannten Sensortypen zutreffen.

Das Inkubationssystem kann zusätzlich eine Regeleinrichtung umfassen, die derart ausgebildet ist, den besagten Parameter auf Grundlage eines zugehörigen Messwerts des Parameters von dem Sensor oder den Sensoren zu regeln.

Die Regeleinrichtung kann in Form einer zentralen Recheneinheit in der Inkubationsvorrichtung ausgebildet sein, wobei die Regeleinrichtung insbesondere Teil der Inkubationsvorrichtung ist. Die Regeleinrichtung kann konfiguriert sein, die zu regelnden Parameter innerhalb des gesamten Inkubationssystems zu regeln.

Hierzu empfängt die Regeleinrichtung Messwerte von den Sensoren in den Inkubationskammern und ist selbst mit den zu steuernden Komponenten des Inkubationssystems, beispielsweise Pumpen oder Heizelementen verbunden, um deren Funktion derart zu steuern, dass die Parameter an einen vorgegebenen Sollwert angepasst werden. Die Verbindung zwischen der Regeleinrichtung und den zu steuernden Komponenten in benachbarten Inkubationskammern kann beispielsweise über die zugehörigen Anschlüsse an den Schnittstellen hergestellt sein.

Alternativ kann die Regeleinrichtung dezentral ausgebildet sein, wobei die Regelung innerhalb jeder Inkubationskammer separat durchgeführt wird. Hierzu erhält jede Regeleinrichtung Sollwerte für die zu regelnden Parameter und regelt die Funktion aktiver Komponenten in derselben Inkubationskammer auf Grundlage von Sensor-Messwerten, wobei die zugehörigen Sensoren ebenfalls in derselben Kammer wie die entsprechende Regeleinrichtung angeordnet ist.

Das Inkubationssystem kann weiterhin eine dritte Inkubationskammer umfassen, wobei die dritte Inkubationskammer eine Verbindungsöffnung mit einer Verbindungseinrichtung umfasst, wobei die erste Inkubationskammer oder die zweite Inkubationskammer eine zweite Verbindungsöffnung mit einer daran angeordneten Verbindungseinrichtung umfasst, und wobei die Verbindungseinrichtung der dritten Inkubationskammer mit der Verbindungseinrichtung der zweiten Verbindungsöffnung der ersten Inkubationskammer oder der zweiten Inkubationskammer verbunden ist.

Je nachdem, ob die dritte Inkubationskammer an der ersten Inkubationskammer oder der zweiten Inkubationskammer angebracht ist, ist deren Anordnung parallel oder in Serie zur ersten Inkubationskammer. Die Anbringung einer dritten Inkubationskammer erhöht zusätzlich die Flexibilität des Inkubationssystems. Beispielsweise kann in der dritten Inkubationskammer eine Kammer-Versorgungseinrichtung angeordnet sein, während in der zweiten Kammer eine Proben-Versorgungseinrichtung angeordnet ist. Der modulare Aufbau des Inkubationssystems erlaubt also mehr als zwei Inkubationskammern und damit eine hohe Flexibilität, indem ein Inkubationssystem mit bestimmten Geräten individuell komponiert werden kann. Gleichzeitig ist es möglich, eine kompakte Bauweise des Inkubationssystems zu erreichen. Einerseits haben unterschiedliche Peripheriegeräte einen individuellen Raumbedarf, an den die einzelnen Inkubationskammern angepasst werden können. Andererseits ist es nicht nötig, stets alle zur Verfügung stehenden Geräte in ein Inkubationssystem zu integrieren, wie das in herkömmlichen Systemen häufig der Fall ist. Stattdessen können nur die benötigten Komponenten montiert und integriert werden, sodass das Inkubationssystem einen geringeren Raumbedarf hat und eine geringere Komplexität aufweist.

Zusätzlich wird mit der vorliegenden Erfindung ein Verfahren zur Analyse einer Probe in einem Inkubationssystem oder einer Inkubationsvorrichtung bereitgestellt, umfassend
Bereitstellen einer zuvor beschriebenen Inkubationsvorrichtung oder eines zuvor beschriebenen Inkubationssystems;
Einbringen eines Probenträgers mit einer Probe in den Probenträgerhalter; und
Durchführen einer Untersuchung an dem Probenträger mit der Untersuchungsvorrichtung.

Das Verfahren im Zusammenspiel mit dem Inkubationssystem bietet jene Vorteile, die auch die Vorrichtung mit sich bringt. Diese sind eine Untersuchung der Probe in einem Inkubationssystem, das speziell für die Untersuchung konzipiert sein kann. Durch die Anordnung der Proben-Versorgungseinrichtung und eventuell weiterer Einrichtungen innerhalb des Inkubationssystem können stabile und optimale Kultivierungsbedingungen für die Probe bereitgestellt werden.

Ein Probenträger ist hierbei eine Plattform für die Untersuchung von Zellen. Der Probenträger kann insbesondere eine plane Unterseite besitzen, was für mikroskopische Untersuchungen durch diese Unterseite hindurch, vor allem im Fall inverser Mikroskopie, besonders von Vorteil ist. Weiterhin besteht der Probenträger vorzugsweise aus einem transparenten Material, beispielsweise aus Kunststoffen wie COC (Cyclo-Olefin Copolymer), COP (Cyclo-Olefin Polymer), PC (Polycarbonat), PS (Polystyrol), PE (Polyethylen), PMMA (Polymethymetacrylat) oder einen transparenten Thermoplast oder ein Elastomer oder einer Mischung davon.

Durch die Verwendung der genannten Materialien und Verfahren können die Probenträger kostengünstig, sowie in großer Stückzahl mit konstanter Qualität produziert werden. Dies liegt daran, dass der Spritzguss mit Kunststoffen ein etabliertes und verlässliches Verfahren ist und insbesondere im Fall der genannten Kunststoffe anwendbar ist. Die Verwendung eines transparenten Kunststoffs ist insbesondere vorteilhaft, um optische Untersuchungen im Probenträger vornehmen zu können, beispielsweise via Mikroskopie.

Der Probenträger kann einen Kanal und ein Reservoir umfassen, wobei das Reservoir mit dem Kanal verbunden sein kann. Beispielsweise ist die Probe in dem Reservoir angeordnet und das Nährmedium strömt durch den Kanal und kann daher die Probe mit Nährstoffen versorgen.

Das Einbringen des Probenträgers kann auch umfassen, dass dieser in dem Probenträgerhalter gehaltert wird. Dazu kann der Probenträgerhalter eine entsprechende Halterung wie beispielsweise einen Rahmen für den Probenträger umfassen, worin der Probenträger schließlich festgeschraubt oder anders fixiert wird.

Das Verfahren kann umfassen, dass während der Durchführung der Messung ein Parameter von Temperatur, Luftfeuchtigkeit und Gaskonzentration in der ersten Inkubationskammer und/oder der zweiten Inkubationskammer eingestellt wird. Der besagte Parameter kann insbesondere geregelt werden.

Auch hier treffen die gemachten Aussagen zu den Vorteilen einer Regelung der Parameter mit einer Regeleinrichtung zu. Diese sind unter anderem, dass der Parameter ohne notwendige Kontrolle geregelt wird und über lange Zeiträume stabil und präzise eingestellt werden kann.

Das beschriebene Verfahren kann weiterhin umfassen, dass eine Flüssigkeit in der zweiten Inkubationskammer zumindest zeitweise auf eine höhere Temperatur temperiert wird als in der ersten Inkubationskammer.

Da die Untersuchungsvorrichtung zusammen mit dem Probenträger in der ersten Inkubationskammer angeordnet ist, ist die Flüssigkeit in einem Bereich der Untersuchung kühler als in der zweiten Inkubationskammer. Dadurch kann eine Blasenbildung in der Flüssigkeit im Bereich der Untersuchung zusätzlich unterdrückt werden.

Weitere Merkmale und Vorteile werden nachfolgend anhand der beispielhaften Figuren erläutert. Dabei zeigen:
- Figur 1: eine schematische Inkubationsvorrichtung als Schrägansicht;
- Figuren 2A bis 2C: unterschiedliche schematische Ausführungsformen einer Verbindungseinrichtung der Inkubationsvorrichtung;
- Figur 3A: ein schematisches Inkubationssystem gemäß einer ersten Ausführungsform im Querschnitt;
- Figur 3B: ein schematisches Inkubationssystem gemäß einer zweiten Ausführungsform im Querschnitt;
- Figur 4: ein schematisches Inkubationssystem gemäß einer weiteren Ausführungsform im Querschnitt.

Im Folgenden und in den Figuren werden in den verschiedenen Ausführungsbeispielen, sofern nicht anders spezifiziert, die gleichen Bezugszeichen für gleiche oder entsprechende Elemente verwendet.

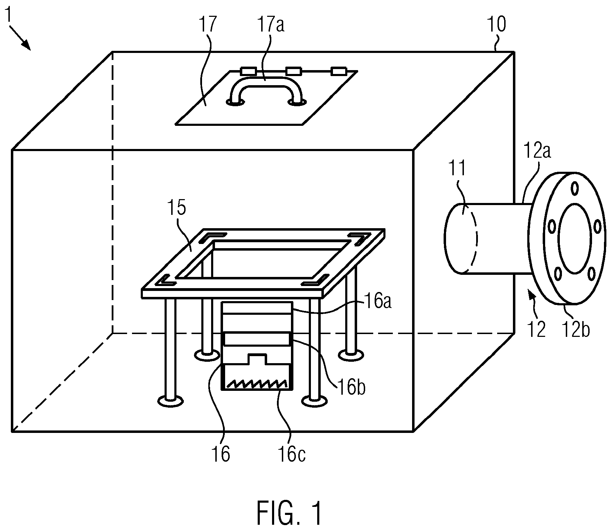

Figur 1 zeigt eine Inkubationsvorrichtung 1 gemäß einer ersten Ausführungsform in einer Schrägansicht. Die Inkubationsvorrichtung 1 umfasst eine erste Inkubationskammer 10. Die Außenwand der Inkubationskammer 10 kann beispielsweise aus Glas oder Acrylglas gefertigt sein, damit die Außenwand transparent ist. Grundsätzlich kommen allerdings auch andere im Zusammenhang mit Inkubationsvorrichtungen übliche Materialien wie Bleche aus Aluminium, Weißmetallen oder Edelstahl, Isolationsschichten aus expandiertes Polystyrol (Styropor), Schaumstoff oder anderen isolierenden Materialien, sowie spritzgegossene Formteile aus Thermoplasten wie Polycarbonat oder Polyvinylchlorid (PVC) in Betracht. Die Außenwände bilden dabei das Gehäuse der Inkubationskammer 10.

In der oberen Seite der Außenwand befindet sich eine Einlassöffnung 17, die im gezeigten Fall in Form einer Klappe mit Scharnieren und einem Griff 17a ausgebildet ist. Dies ist ein einfach zu realisierendes und zu verwendendes Beispiel für eine Einlassöffnung 17. Die Einlassöffnung 17 kann beispielsweise verwendet werden, um eine Probe in den Innenraum der Inkubationsvorrichtung 1, beziehungsweise der ersten Inkubationskammer 10 einzubringen. Es können auch andere Objekte in die Inkubationskammer 10 eingebracht oder daraus entnommen werden oder andere Arbeiten in der Inkubationskammer 10 durchgeführt werden Beispielsweise kann ein Schlauch innerhalb der Inkubationskammer 10 verlegt werden. Die Einlassöffnung 17 kann auch an einer anderen Stelle der Inkubationsvorrichtung 1 angebracht sein und ist auch nicht auf die Form einer Klappe mit einem Griff 17a beschränkt.

Weiterhin umfasst die Inkubationsvorrichtung 1 eine Verbindungsöffnung 11 die an einer anderen Stelle der Außenwand ausgebildet ist als die Einlassöffnung 17. An der Verbindungsöffnung 11 ist eine Verbindungseinrichtung 12 angebracht, die derart ausgebildet ist, dass daran eine zweite Inkubationskammer, die ebenfalls eine Verbindungsöffnung mit einer Verbindungseinrichtung umfasst, angebracht werden kann. Dabei müssen die Verbindungseinrichtung 12 der ersten Inkubationskammer 10 und der zweiten Inkubationskammer zueinander passen oder entsprechende Gegenstücke sein, damit eine stabile Verbindung erzeugt werden kann. Im gezeigten Beispiel umfasst die Verbindungseinrichtung 12 ein Rohrstück 12a mit einem Flansch 12b. Eine zweite Verbindungseinrichtung der zweiten Inkubationskammer kann ebenfalls einen Flansch umfassen, sodass beide Verbindungseinrichtungen zusammengeflanscht und verschraubt werden können.

Für den Fall, dass die Inkubationsvorrichtung 1 verschlossen werden soll, kann ein Blindflansch auf die Verbindungseinrichtung 12 angebracht werden. Dieser Blindflansch entspricht einer zuvor beschriebenen Verschlussvorrichtung.

Die Verbindungseinrichtung 12 kann grundsätzlich aus dem gleichen Material wie die Außenwand der Inkubationskammer 10 bestehen. Allerdings kann es auch von Vorteil sein, wenn die Verbindungseinrichtung 12 aus einem anderen Material besteht wie die Außenwand der Inkubationskammer 10. Beispielsweise kann es für eine Verbindung mit einem Flansch 12b von Vorteil sein, wenn der Flansch 12b selbst aus einem Metall wie Stahl besteht oder dieses zumindest umfasst. Bei einer Verbindung mit Metallschrauben widersteht ein Flansch 12b aus Metall einer höheren Belastung als Kunststoff oder Glas. Dies verringert die Wahrscheinlichkeit von Beschädigungen an der Inkubationsvorrichtung durch hohe, gegebenenfalls punktuelle, Belastungen der Kopplungseinrichtung 12.

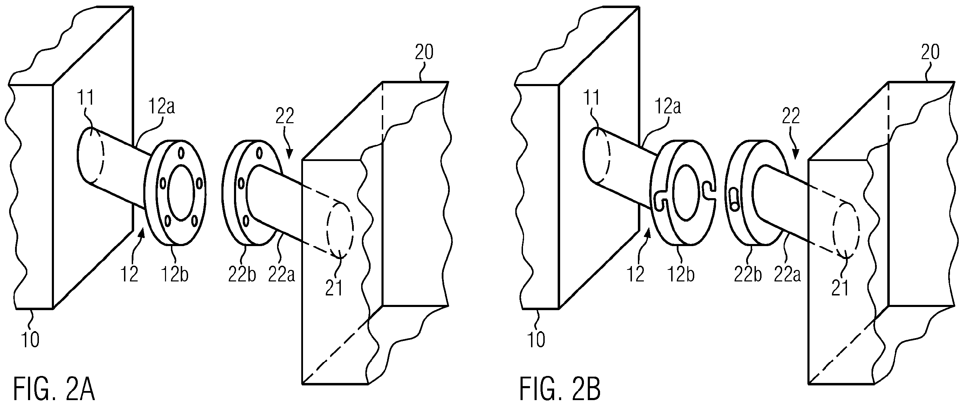

Die Verbindungseinrichtung 12 ist nicht auf das gezeigte Beispiel beschränkt und weitere Beispiele werden nachfolgend mit Bezug auf Figuren 2A bis 2C erläutert.

Die Inkubationsvorrichtung 1 der Figur 1 umfasst weiterhin einen Probenträgerhalter 15 und eine Untersuchungsvorrichtung 16, die im Inneren der Inkubationskammer 10 angeordnet sind. Der Probenträgerhalter 15 ist im vorliegenden Beispiel in Form eines Tisches ausgebildet, auf dem ein Probenträger platziert und gehaltert werden kann. In der Unterseite des Probenträgerhalters 15 befindet sich eine Aussparung in dem Tisch, durch die hindurch eine Untersuchung mit einer Untersuchungsvorrichtung 16 durchgeführt werden kann.

Die Untersuchungsvorrichtung 16 ist im vorliegenden Fall eine optische Vorrichtung, beziehungsweise eine Bilderfassungsvorrichtung, in Form eines inversen Mikroskops. Dieses Mikroskop umfassen ein Objektiv 16a, ein Okular 16b und eine Kamera 16c zur Aufnahme von Bildern. Die drei genannten Komponenten werden von einem Tubusrohr umgeben, um eine höhere Stabilität zu gewährleisten und Störquellen wie Streulicht nicht auf die Kamera 16c auftreffen zu lassen. Das von der Kamera 16c erzeugte Bild kann durch eine entsprechende elektronische Verbindung aus der Inkubationskammer 10 zu einer Verarbeitungseinrichtung oder einer Bedienschnittstelle eines Benutzers (nicht gezeigt), wie einem Computer, geführt werden.

An dieser Stelle zeigt sich neben der erhöhten Flexibilität noch ein weiterer Vorteil der beschriebenen Inkubationsvorrichtung 1. Die optischen Komponenten, insbesondere das Objektiv 16a des beschriebenen Mikroskops befinden sich mit dem Probenträger und damit der Probe in der Inkubationskammer 10 der Inkubationsvorrichtung 1 und sind somit denselben Bedingungen ausgesetzt. Daher gibt es zwischen dem Probenträger und dem Objektiv 16a keinen Unterschied in der Temperatur oder der Luftfeuchtigkeit. Hierdurch wird vermieden, dass Flüssigkeit an dem Objektiv 16a oder dem Probenträger kondensiert und die Untersuchung verfälscht. Dies könnte gegebenenfalls passieren, falls das Objektiv 16a in Kontakt mit dem Probenträger ist und dabei ein Temperaturgradient zwischen dem Probenträger und dem Objektiv 16a besteht. Dieser Vorteil trifft auch zu, wenn es sich bei der Untersuchungsvorrichtung 16 nicht um ein inverses Mikroskop, sondern um eine andere Vorrichtung, beispielsweise für die Messung einer Impedanz, handelt.

Figuren 2A bis 2C zeigen unterschiedliche Ausführungsformen von Verbindungseinrichtungen 12, 22, mit der die erste Inkubationskammer 10 der Inkubationsvorrichtung 1 und eine zweite Inkubationskammer 20 miteinander verbunden werden können.

Figur 2A zeigt einen Ausschnitt der ersten Inkubationskammer 10 mit der Verbindungsöffnung 11 und einer daran abgebrachten Verbindungseinrichtung 12. Diese Verbindungseinrichtung 12 umfasst ausgehend von der Verbindungsöffnung 11 ein Rohrstück 12a, an dessen von der ersten Inkubationskammer 10 abgewandten Ende ein Flansch 12b angebracht ist. Eine zweite Inkubationskammer 20, die mit der ersten Inkubationskammer 10 verbunden werden soll, weist eine baugleiche Verbindungseinrichtung 22 mit einem an der Verbindungsöffnung 21 angebrachten Rohrstück 22a und einem an dessen Ende angebrachten Flansch 22b auf. Die beiden Flansche können miteinander verschraubt, mit Schrauben oder Schraubbolzen (nicht dargestellt), und somit verbunden werden.

Um eine verbesserte Abdichtung und thermische Isolierung der verbundenen Verbindungseinrichtungen 12, 22 gegenüber einer Umgebung zu erreichen, kann zwischen die beiden Flansche zusätzlich ein Dichtungselement, beispielsweise ein Dichtring aus Gummi oder Kupfer, angeordnet sein. Solche Dichtungen werden zum Beispiel im Zusammenhang mit Vakuumvorrichtungen verwendet.

Eine weitere Form einer Verbindungseinrichtung 12, 22 wird mit Bezug auf Figur 2B erläutert. Diese unterscheidet sich von der Verbindungseinrichtung der Figur 2A dahingehend, dass anstelle eines Flansches ein Bajonettverschluss angebracht ist. Dabei umfasst die Verbindungseinrichtung 12 der ersten Inkubationskammer 10 ein Rohrstück 12a und an dessen von der Inkubationskammer 10 abgewandten Ende ein drehbares Außenstück 12b mit zwei gegenüberliegenden Längsschlitzen, an deren Ende sich rechtwinklig ein kurzer Querschlitz anschließt. Die Verbindungseinrichtung 22 einer zweiten Inkubationskammer 20 umfasst dabei das zugehörige Gegenstück. An der Verbindungsöffnung 21 ist ein Rohrstück 22a angebracht, an dessen Ende ein Innenstück 22b angeordnet ist. Dieses Innenstück 22b wird in das Außenstück 12b geschoben und umfasst einen Knopf, der in den Längsschlitz geführt wird. Durch Drehen des Außenstücks 12b wird eine feste Verbindung erreicht, indem der Knopf in den Querschlitz einrastet.

Die Anordnung der Verbindungseinrichtung kann auch umgekehrt sein, in dem Sinne, dass die Verbindungseinrichtung 12 der ersten Inkubationskammer 10 Innenstück umfasst und die Verbindungseinrichtung 22 der zweiten Inkubationskammer 20 entsprechend das Außenstück umfasst. Weiterhin kann auch hier ein Dichtungselement verwendet werden. Ein Vorteil eines Bajonettverschlusseses gegenüber einer Flanschverbindung ist, dass keine Werkzeuge und/oder Schrauben benötigt werden.

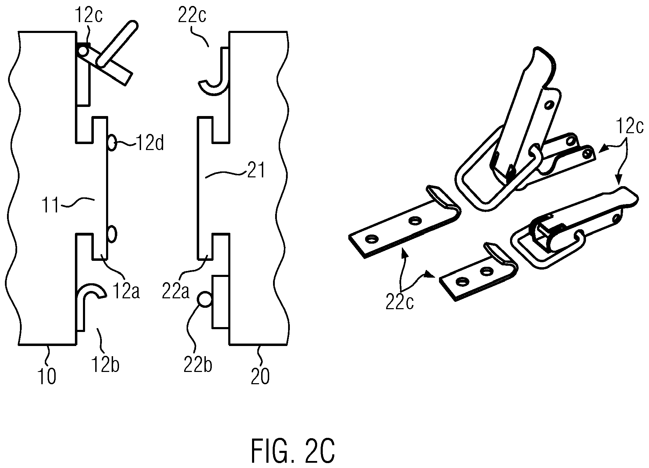

Figur 2C zeigt eine weitere Ausführungsform besagter Verbindungseinrichtungen im Querschnitt. Hierbei umfasst die Verbindungseinrichtung 12 der ersten Inkubationskammer 10 zunächst ein Rohrstück 12a an der Verbindungsöffnung 11. Zudem befinden sich an der Außenseite der ersten Inkubationskammer 10, in der auch die Verbindungsöffnung 11 angeordnet ist, in der unteren Hälfte ein Haken 12b und in der oberen Hälfte ein Spannverschluss 12c. An der zugewandten Außenseite der zweiten Inkubationskammer 20 befinden sich neben einem Rohrstück 22a, das an der Verbindungsöffnung 21 angebracht ist, in der unteren Hälfte eine Querstange 22b und in der oberen Hälfte ein Haken 22c. Wenn die Verbindungseinrichtungen der ersten Inkubationskammer 10 und der zweiten Inkubationskammer 20 verbunden werden sollen, wird zunächst die Querstange 22b in den Haken 12b an der Außenseite der ersten Inkubationskammer 10 eingehängt. Anschließend wird der Haken 22c an der Außenseite der zweiten Inkubationskammer 20 in eine Öse des Spannverschlusses 12c eingehängt und der Spannverschluss 12c schließlich festgezogen. Dadurch werden die beiden Rohrstücke 12a, 22a aufeinandergepresst und die beiden Inkubationskammern 10, 20 verbunden.

Eine Detailansicht des Spannverschlusses 12c zusammen mit dem Haken 22c ist auch in der Figur 2C gezeigt. Beim Verbinden der Verbindungsrichtungen wird wie beschrieben der Haken 22c in die Öse des Spannverschlusses 12c eingehängt und schließlich der Spannverschluss 12c festgezogen.

Ein zwischen den beiden Rohrstücken 12a, 22a angeordnetes Dichtungselement 12d kann die Dichtigkeit der Verbindung weiter verbessern.

Alle drei gezeigten Beispiele für eine Verbindungseinrichtung haben als gemeinsame Vorteile eine einfache Handhabbarkeit und Reversibilität. Die Verbindung kann mehrmals hergestellt und wieder aufgelöst werden, ohne dass Ersatzteile benötigt werden, weil die Verbindung zerstörungsfrei lösbar ist.

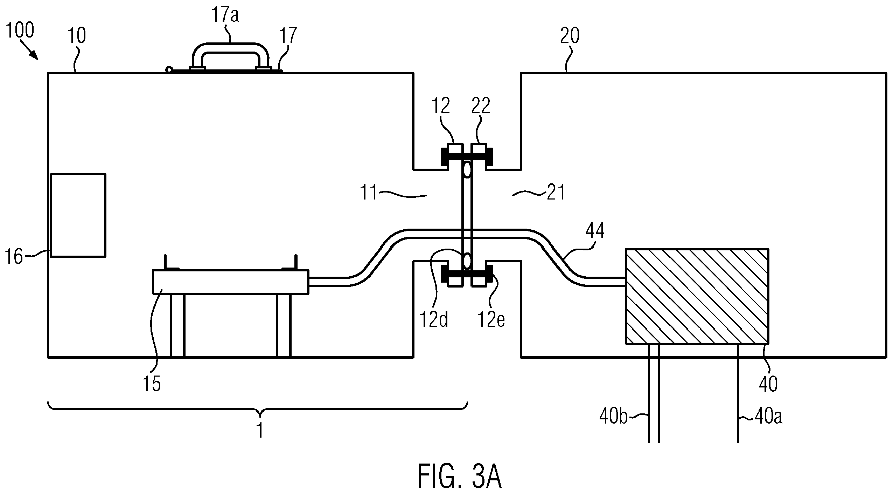

Figur 3A zeigt ein Inkubationssystem 100 gemäß einer ersten Ausführungsform als schematischen Querschnitt. Das Inkubationssystem 100 umfasst eine Inkubationsvorrichtung 1 mit einer ersten Inkubationskammer 10, sowie eine zweite Inkubationskammer 20, wobei die beiden Inkubationskammern durch ihre Verbindungseinrichtungen 12, 22 miteinander verbunden sind. Die Verbindungseinrichtungen 12, 22 können beispielsweise, aber nicht zwingend, wie in Figur 2A erläutert, eine Flanschverbindung ergeben. Die Flansche sind dabei mit Schrauben 12e verschraubt und insbesondere eine gasdichte Verbindung bildend. Zwischen den Kopplungseinrichtungen 12, 22 ist zudem ein Dichtungselement in Form eines Dichtrings 12d zur Verbesserung der Dichtigkeit und thermischen Isolierung der Verbindung angeordnet. Der Dichtring 12d kann beispielsweise aus Gummi oder einem anderen elastischen Material bestehen.

In der ersten Inkubationskammer 10 sind ein Probenträgerhalter 15 und eine Untersuchungsvorrichtung 16 angeordnet. Dabei ist die Untersuchungsvorrichtung 16 lediglich schematisch dargestellt. Beispielsweise kann es sich um ein Impedanzmesssystem handeln. Hierfür müssten Elektroden zur Messung der Impedanz einer biologischen Probe in einen Probenträger eingesetzt werden, sobald der Probenträger in dem Probenträgerhalter 15 gehaltert ist. Bezüglich weiterer Details oder Ausführungsformen der Untersuchungsvorrichtung 16 wird auf die vorstehenden Erläuterungen verwiesen.

Ebenso umfasst die erste Inkubationskammer 10 eine vorstehend beschriebene Einlassöffnung 17 mit einem Griff 17a.

Innerhalb der zweiten Inkubationskammer 20 befindet sich eine Proben-Versorgungseinrichtung 40. Diese besitzt eine Stromversorgung 40a und einen Eingang 40b für Gase und Flüssigkeiten. Die Proben-Versorgungseinrichtung 40 kann aus den eingeführten Gasen und Flüssigkeiten eine Nährmedium mit einer definierten Temperatur und Gaskonzentration erzeugen. Diese Nährmedium wird über eine Verbindung wie einen Schlauch 44 an den Probenträgerhalter 15 in der ersten Inkubationskammer 10 geleitet. Dabei führt der Schlauch 44 durch die Verbindungsöffnungen 11, 21 der ersten Inkubationskammer 10 und der zweiten Inkubationskammer 20 hindurch. Damit die Nährmedium an eine Probe geleitet wird, können im Probenträgerhalter 15 Ventile vorhanden sein, sodass beim Haltern eines Probenträgers die Nährmedium in den Probenträger strömen kann. Alternativ kann der Schlauch 44 auch nach dem Haltern des Probenträgers direkt an dem Probenträger befestigt werden.

Es versteht sich, dass für das Nährmedium ein geschlossener Flüssigkeitskreislauf vorliegen muss. Hierzu wird die Nährmedium vom Probenträgerhalter 15 zur Proben-Versorgungseinrichtung 40 zurückgeführt. Dies kann über einen zweiten Schlauch bewerkstelligt werden, der in der Figur nicht gezeigt ist.

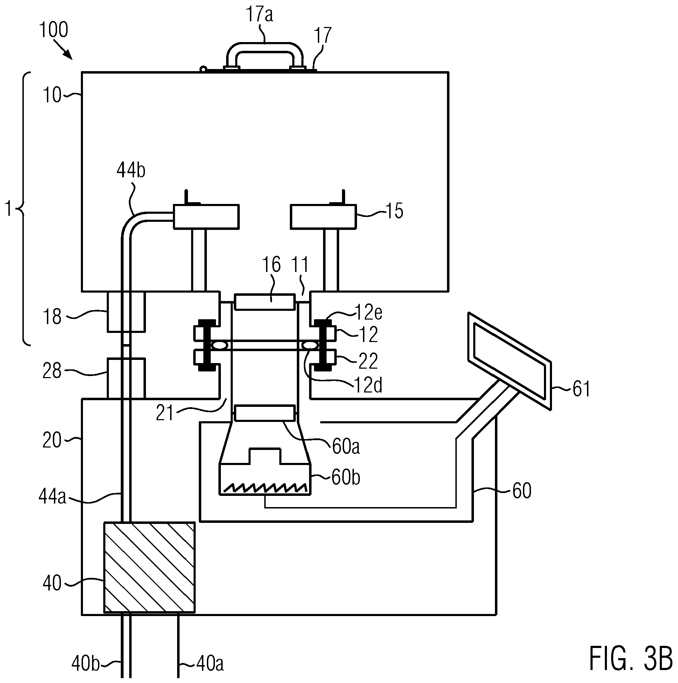

Ein Inkubationssystem 100 umfassend eine beschriebene Inkubationsvorrichtung 1 und eine zweite Inkubationskammer 20 gemäß einer weiteren Ausführungsform ist in Figur 3B dargestellt. Dabei sind die beiden Inkubationskammern mittels ihrer jeweiligen Verbindungseinrichtung 12, 22 miteinander verbunden. Die Verbindungseinrichtungen können insbesondere, wie in den vorstehenden Ausführungsbeispielen beschrieben, eine Flanschverbindung bilden, wobei die beiden Flansche mit Schrauben 12e zusammengehalten werden und ein zwischen den Flanschen angeordnetes Dichtungselement 12d für eine bessere Isolation des Inkubationssystems 100 gegenüber der Umgebung sorgt.

Dabei umfasst die Inkubationsvorrichtung 1 zunächst eine erste Inkubationskammer 10, in der ein Probenträgerhalter 15 und eine Untersuchungsvorrichtung 16 in Form eines Objektivs angeordnet sind. Darüber hinaus verfügt die Inkubationskammer 10 über eine Einlassöffnung 17 mit einem Griff 17a an dessen Oberseite und eine Verbindungsöffnung 11 an dessen Unterseite.

Die zweite Inkubationskammer 20 umfasst in ihrem Inneren weitere Bestandteile eines Mikroskops 60, das durch das beschriebene Objektiv komplettiert wird. Die besagten weiteren Bestandteile sind ein Okular 60a, eine Kamera 60b mit einem Sensor zur Bilderzeugung und ein Stativ (nicht gezeigt). Außerhalb der zweiten Inkubationskammer 20 kann das Mikroskop 60 darüber hinaus über eine Anzeigevorrichtung 61 verfügen, mit der ein von der Kamera 60b erzeugtes Bild einem Benutzer angezeigt wird.

Weiterhin ist ein der zweiten Inkubationskammer 20 eine Proben-Versorgungseinrichtung 40 vorgesehen, die ausgebildet ist, ein Gas und/oder eine Flüssigkeit für eine in dem Probenträgerhalter 15 angeordnete Probe bereitzustellen. Dabei kann die Kammer-Versorgungseinrichtung 40 beispielsweise eine Pumpe umfassen, die eine Nährflüssigkeit mit einer bestimmten Temperatur und/oder einer bestimmten Gaskonzentration an die Probe pumpt. Dies erfolgt im gezeigten Beispiel mit einer Leitung wie einem Schlauch 44a, 44b, der von der Proben-Versorgungseinrichtung 40 über die Verbindung der Schnittstellen 18, 28 der ersten Inkubationskammer 10 und der zweiten Inkubationskammer 20 mit dem Probenträgerhalter 15 verbunden ist. Bezüglich der Versorgung einer Probe mit der Nährmedium durch die Proben-Versorgungseinrichtung 40 und weitere Eigenschaften der Proben-Versorgungseinrichtung 40 gelten die gleichen Erwägungen wie im mit Bezug auf Figur 3A gezeigten Ausführungsbeispiel. Insbesondere umfasst die Proben-Versorgungseinrichtung 40 eine Stromversorgung 40a und einen Eingang 40b. Über diesen Eingang 40b können zum Beispiel Flüssigkeiten oder andere Bestandteile für die Bereitstellung der Nährlösung zugeführt werden.

Neben den Verbindungseinrichtungen 12, 22 sind auch Schnittstellen 18, 28 der ersten Inkubationskammer 10 und der zweiten Inkubationskammer 20 miteinander verbunden, wodurch ein geschlossener Flüssigkeitsweg über die Schläuche 44a und 44b von der Proben-Versorgungseinrichtung 40 zum Probenträgerhalter 15 hergestellt wird. Dabei sind die beiden Abschnitte des Schlauchs 44a, 44b an der Schnittstelle 28 beispielsweise in Form einer Luerlock-Verbindung flüssigkeitsdicht miteinander verbunden.

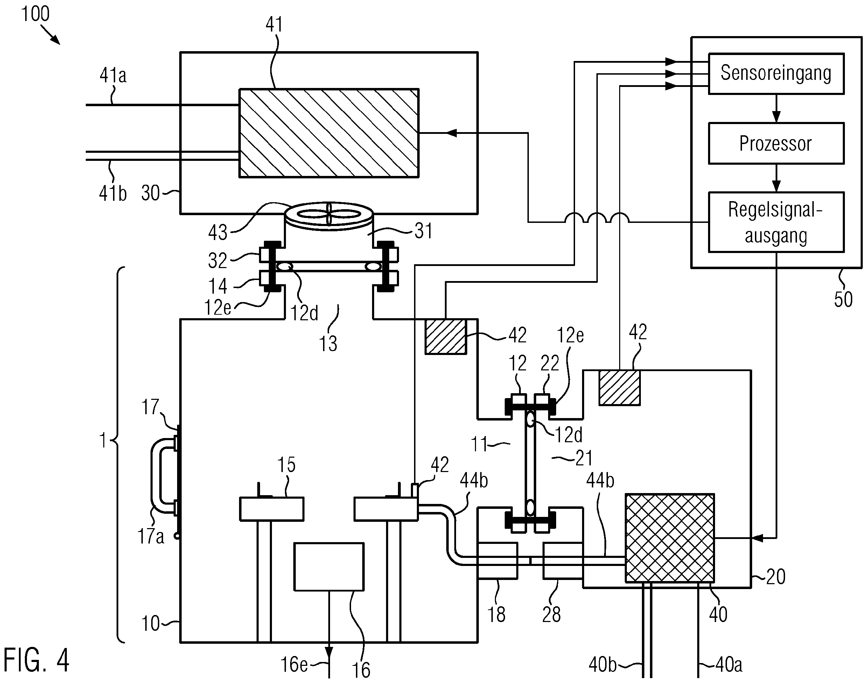

In Figur 4 ist ein Inkubationssystem 100 umfassend eine Inkubationsvorrichtung 1 und zwei weitere Inkubationskammern, also insgesamt drei Inkubationskammern, dargestellt. Wie bereits in den vorangegangenen Ausführungsbeispielen sind in der ersten Inkubationskammer 10 eine Untersuchungsvorrichtung 16 und einen Probenträgerhalter 15 angeordnet, wobei die Untersuchungsvorrichtung 16 hier nur schematisch dargestellt ist. Hierbei kann es sich beispielsweise um ein beschriebenes Bilderfassungssystem oder Impedanzmesssystem handeln. Messergebnisse der Untersuchungsvorrichtung können über die Leitung 16e aus der Kammer beispielsweise an eine Analysevorrichtung (nicht gezeigt) übermittelt werden. Eine Einlassöffnung 17 in Form einer Klappe und mit einem Griff 17a ist seitlich an der der ersten Inkubationskammer 10 angebracht.

Das Inkubationssystem 100 umfasst auch eine zweite Inkubationskammer 20, deren Verbindungseinrichtung 22 mit der Verbindungeinrichtung 12 der ersten Inkubationskammer 10 verbunden ist. Diese Verbindungseinrichtungen 12, 22 können in Form von Flanschen ausgebildet und dabei mittels Schrauben 12e oder Schraubbolzen verschraubt sein. Insgesamt sind die Verbindungseinrichtungen 12, 22 nur vereinfacht gezeigt und nicht auf eine Schraubverbindung mit Flanschen beschränkt. Es wird auf die vorstehenden Details hierzu verwiesen.

Im Inneren der zweiten Inkubationskammer 20 ist eine Proben-Versorgungseinrichtung 40 angeordnet. Diese besitzt einen Flüssigkeitseingang 40b und eine Stromversorgung 40a. Die zu diesen Eingängen führenden Verbindungen können durch Durchlassöffnungen in einer Außenwand der zweiten Inkubationskammer 20 von außen vorliegen. Ein Ausgang der Proben-Versorgungseinrichtung 40 ist in Form eines Schlauchs 44a ausgebildet, der eine Nährmedium mit den der Proben-Versorgungseinrichtung 40 bereitgestellter Temperatur und Gaskonzentration führen kann. Dieser Schlauch 44a führt zu einer Schnittstelle 28 der zweiten Inkubationskammer 20.

Auch die erste Inkubationskammer 10 verfügt über eine Schnittstelle 18, die mit der Schnittstelle 28 der zweiten Inkubationskammer 20 verbunden ist. Damit entsteht ein geschlossener Flüssigkeitsweg für besagtes Nährmedium von der zweiten Inkubationskammer 20 in die erste Inkubationskammer 10, indem der Schlauch 44a mit dem Schlauch 44b an den Schnittstellen 18 und 28 verbunden wird, beispielsweise über eine Luerlock-Verbindung. In der ersten Inkubationskammer 10 verbindet der Schlauch 44b die Schnittstelle 18 mit dem Probenträgerhalter 15. Der Probenträgerhalter 15 kann über ein Ventil verfügen, sodass die Nährmedium vom Probenträger in einen eingesetzten Probenträger strömen kann. Alternativ kann der Schlauch auch direkt mit einem Probenträger verbunden werden, sobald dieser in dem Probenträgerhalter 15 gehaltert ist.

Darüber hinaus verfügt das Inkubationssystem 100 noch über eine dritte Inkubationskammer 30, wobei die zweite Verbindungseinrichtung 14 der ersten Inkubationskammer 10 mit einer Verbindungseinrichtung 32 der dritten Inkubationskammer 30 verbunden ist. Somit sind die Innenräume der ersten Inkubationskammer 10 und der dritten Inkubationskammer 30 durch die Verbindungsöffnungen 11 und 31 verbunden. Diese Verbindung kann in der gleichen Weise ausgestaltet sein wie zwischen der ersten Inkubationskammer 10 und der zweiten Inkubationskammer 20, kann aber auch in unterschiedlicher Weise ausgestaltet sein. In der dritten Inkubationskammer 30 ist eine Kammer-Versorgungseinrichtung 41 angeordnet. Diese ist vor allem ausgebildet Luftfeuchtigkeit und Gaskonzentration innerhalb des Inkubationssystems 100 einzustellen. Hierzu hat die Inkubationskammer-Versorgungseinrichtung 41 eine Stromversorgung 41a und einen Wasseranschluss 41b als Eingänge, die von außen durch vorgesehene Durchlassöffnungen in die dritte Inkubationskammer 30 hineingeführt sind. Die Kammer-Versorgungseinrichtung 41 kann zudem über ein Heizelement verfügen, um Luft auch entsprechen erwärmen zu können. Insgesamt ist die Kammer-Versorgungseinrichtung 41 ausgebildet, Luft mit einer bestimmten Temperatur und Luftfeuchtigkeit zu erzeugen und diese an den Innenraum die dritte Inkubationskammer 30 abzugeben.

In der dritten Inkubationskammer 30 ist auch ein Luftverteiler, insbesondere ein Ventilator 43 angeordnet. In diesem Fall ist der Ventilator 43 an der Verbindungsöffnung 31 der dritten Inkubationskammer 30 angebracht. Der Ventilator 43 verteilt die Luft der dritten Inkubationskammer innerhalb des gesamten Inkubationssystems 100 und erreicht dadurch eine schnelle Angleichung der Umgebungsbedingungen in allen drei Inkubationskammern 10, 20 und 30.

Das Inkubationssystem 100 umfasst eine Mehrzahl von Sensoren 42, die ausgebildet sind, mindestens einen Parameter von Temperatur, Luftfeuchtigkeit und Gaskonzentration zu bestimmen. Die Bestimmung kann insbesondere zeitaufgelöst sein. Jeweils einer dieser Sensoren 42 befindet sich in der ersten Inkubationskammer 10 und der zweiten Inkubationskammer 20, um die Umgebungsbedingungen zu erfassen und einen Messwert oder ein korrespondierendes Messsignal auszugeben. Ein dritter Sensor 42 befindet sich auf dem Probenträgerhalter 15 und damit in unmittelbarer Nähe zu einer darin angeordneten Probe. Dieser Sensor ist damit in der Lage, die Bedingungen in der Nähe der Probe zu erfassen. Insbesondere ist dieser Sensor 42 in der Lage, die Parameter der von der Proben-Versorgungseinrichtung 40 bereitgestellten Nährmedium zu ermitteln, sodass Messdaten dieses Sensors für eine Regelung der Proben-Versorgungseinrichtung 40 herangezogen werden können.

Die Messwerte oder Messsignale der Sensoren 42 werden an einen Sensoreingang einer Regeleinrichtung 50 übertragen, beispielsweise über eine elektrische Verbindung. Diese elektrische Verbindung ist in diesem Fall eine elektrische Durchführung durch die Außenwand der Inkubationskammern. Alternativ kann die elektrische Verbindung auch an einer Schnittstelle der Inkubationskammern ausgebildet sein. Es ist auch denkbar, dass die Übertragung durch eine drahtlose Verbindung hergestellt wird, beispielsweise über Funk, Bluetooth oder WLAN. Die Regeleinrichtung 50 bekommt als weiteren Input einen Sollwert für den zu regelnden Parameter. Dieser Parameter kann beispielsweise von einem Benutzer über eine Benutzer-Schnittstelle in die Regeleinrichtung 50 eingegeben werden.

Die vom Sensoreingang empfangenen Werte werden an den Prozessor der Regeleinrichtung 50 übergeben. Der Prozessor ist konfiguriert, aus den empfangenen Werten und dem Sollwert ein Fehlersignal zu berechnen, das zu der Abweichung zwischen dem vom Sensor gemessenen Wert und dem Sollwert korrespondiert. Dieses Fehlersignal dient als Regelsignal. Je nach zu regelndem Parameter wird das Regelsignal von dem Regelsignalausgang an die Inkubationskammer-Versorgungseinrichtung 41 und/oder die Proben-Versorgungseinrichtung 40 übertragen. Dieses Regelsignal hat zur Folge, dass die genannten Versorgungeinrichtungen ihre Funktion derart anzupassen, dass der zu regelnde Parameter an den Sollwert angepasst wird. Beispielsweise kann bei einer zu hohen Temperatur im Inkubationssystem 100 eine Heizvorrichtung der Inkubationskammer-Versorgungseinrichtung 41 heruntergefahren werden. Die beschriebene Regelung basiert damit auf einer feedbackbasierten Regelschleife und kann kontinuierlich durchgeführt werden.

Die Regeleinrichtung 50 ist als externe Einrichtung gezeigt, allerdings kann sie auch innerhalb des Inkubationssystems 100 vorliegen. Ebenso ist es möglich, dass die Kammer-Versorgungseinrichtung 41 und/oder die Proben-Versorgungseinrichtung 40 die Komponenten der Regeleinrichtung 50 implementiert haben. In diesem Fall würden die Messwerte der Sensoren 42 direkt an die Kammer-Versorgungseinrichtung 41 und/oder die Proben-Versorgungseinrichtung 40 übertragen und dort für die Regelung der entsprechenden Einrichtungen verwendet. Die obigen Erläuterungen zur Regeleinrichtung 50 treffen auch in diesem Fall zu.

Die beschriebenen Ausführungsformen sind nicht als einschränkend zu verstehen. Beispielsweise sind die Kombinationen von Merkmalen nicht auf eine bestimmte Anzahl an Inkubationskammern oder bestimmte Versorgungseinrichtungen des Inkubationssystems 100 gebunden.

## Patentansprüche

1. Inkubationsvorrichtung (1), umfassend:
eine erste Inkubationskammer (10);
wobei in der ersten Inkubationskammer (10) angeordnet sind:
ein Probenträgerhalter (15), und
eine Untersuchungsvorrichtung (16) zur Untersuchung einer Probe, die in dem Probenträgerhalter (15) angeordnet ist,
wobei die erste Inkubationskammer (10) eine Verbindungsöffnung (11) zum Verbinden mit einer zweiten Inkubationskammer (20) und eine Einlassöffnung (17) zum Einbringen der Probe in die erste Inkubationskammer (10) umfasst,
wobei an der Verbindungsöffnung (11) eine Verbindungseinrichtung (12) angeordnet ist, und
wobei die Verbindungseinrichtung (12) mit einer Verbindungseinrichtung (22), die an einer Verbindungsöffnung (21) der zweiten Inkubationskammer (20) angeordnet ist, verbindbar ist.

2. Inkubationsvorrichtung (1) nach Anspruch 1, wobei die Verbindungseinrichtung (12) der ersten Inkubationskammer (10) eine Komponente einer Schraubverbindung, einer magnetischen Verbindung mit Positioniermechanik, eines Bajonettverschlusses und/oder einer Einhängeverbindung, beispielsweise mit einem Spannverschluss, umfasst.

3. Inkubationsvorrichtung (1) nach einem der vorangegangenen Ansprüche, weiterhin umfassend eine Schnittstelle (18) in einer Außenwand der ersten Inkubationskammer (10), wobei die Schnittstelle (18) über einen Anschluss für eine elektrische, gasführende und/oder flüssigkeitsführende Verbindung verfügt.

4. Inkubationsvorrichtung (1) nach Anspruch 4, wobei die Schnittstelle (18) eine Steckverbindung, insbesondere mit einem Dichtungselement, umfasst.

5. Inkubationsvorrichtung (1) nach einem der vorangegangenen Ansprüche, wobei die erste Inkubationskammer (10) eine zweite Verbindungsöffnung (13) aufweist, an der eine zweite Verbindungseinrichtung (14) angeordnet ist.

6. Modulares Inkubationssystem (100), umfassend:
Inkubationsvorrichtung (1) nach einem der vorangegangenen Ansprüche, und
eine zweite Inkubationskammer (20) mit einer Verbindungsöffnung (21),
wobei an der Verbindungsöffnung (21) der zweiten Inkubationskammer (20) eine Verbindungseinrichtung (22) angeordnet ist, und
wobei die Verbindungseinrichtung (12) der ersten Inkubationskammer (10) mit der Verbindungseinrichtung (22) der zweiten Inkubationskammer (20) verbunden ist.

7. Inkubationssystem (100) nach Anspruch 6, weiterhin umfassend eine Proben-Versorgungseinrichtung (40) zur Bereitstellung eines Gases oder einer Flüssigkeit für die in dem Probenträgerhalter (15) angeordnete Probe,
wobei die Proben-Versorgungseinrichtung (40) insbesondere eine Pumpe mit einem Flüssigkeitsreservoir umfasst.

8. Inkubationssystem (100) nach Anspruch 6 oder 7, wobei eine Inkubationsvorrichtung (1) nach einem der Ansprüche 3 oder 4 bereitgestellt wird,
wobei in einer Außenwand der zweiten Inkubationskammer (20) eine Schnittstelle (28) für einen Anschluss einer gasführendenden und/oder flüssigkeitsführenden Verbindung (40a) angeordnet ist, und
wobei die Schnittstelle (18) der ersten Inkubationskammer (10) mit der Schnittstelle (28) der zweiten Inkubationskammer (20) verbunden ist, sodass eine geschlossene gasführende und/oder flüssigkeitsführende Verbindung vorliegt.

9. Inkubationssystem (100) nach einem der Ansprüche 6 bis 8, weiterhin umfassend ein Heizelement, das ausgebildet ist, eine Temperatur in der ersten Inkubationskammer (10) und/oder der zweiten Inkubationskammer (20) einzustellen, und/oder
eine Kammer-Versorgungseinrichtung (41), die ausgebildet ist, eine Luftfeuchtigkeit und/oder Gaskonzentration, insbesondere CO₂- und/oder O₂-Konzentration, in der ersten Inkubationskammer (10) und/oder der zweiten Inkubationskammer (20) einzustellen.

10. Inkubationssystem (100) nach Anspruch 9, wobei das Heizelement ausgebildet ist, die Temperatur in der ersten Inkubationskammer (10) und in der zweiten Inkubationskammer (20) unabhängig voneinander einzustellen, und/oder
wobei die Kammer-Versorgungseinrichtung (41) ausgebildet sind, die Luftfeuchtigkeit und/oder die Gaskonzentration in der ersten Inkubationskammer (10) und in der zweiten Inkubationskammer (20) unabhängig voneinander einzustellen.

11. Inkubationssystem (100) nach einem der Ansprüche 6 bis 10, weiterhin umfassend einen Luftverteiler (43), beispielsweise einen Ventilator, für einen Austausch einer Temperatur, Gaskonzentration und/oder einer Luftfeuchtigkeit zwischen der ersten Inkubationskammer (10) und der zweiten Inkubationskammer (20).

12. Inkubationssystem (100) nach einem der Ansprüche 6 bis 11, wobei die erste Inkubationskammer (10) und/oder die zweite Inkubationskammer (20) gegenüber einer Umgebung thermisch isoliert sind/ist.

13. Inkubationssystem (100) nach einem der Ansprüche 6 bis 12, weiterhin umfassend:
einen Temperatursensor (42), Luftfeuchtesensor (42) und/oder Gaskonzentrationssensor (42) zur Bestimmung mindestens eines Parameters von Temperatur, Luftfeuchtigkeit und/oder Gaskonzentration in der ersten Inkubationskammer (10) und/oder der zweiten Inkubationskammer (20).

14. Inkubationssystem (100) nach Anspruch 13, weiterhin umfassend eine Regeleinrichtung (50), die derart ausgebildet ist, den besagten Parameter auf Grundlage eines zugehörigen Messwerts von dem Sensor (42) oder den Sensoren (42) zu regeln.

15. Inkubationssystem (100) nach einem der Ansprüche 6 bis 14, weiterhin umfassend eine dritte Inkubationskammer (30),
wobei die dritte Inkubationskammer (30) eine Verbindungsöffnung (31) mit einer daran angeordneten Verbindungseinrichtung (32) umfasst,
wobei die erste Inkubationskammer (10) oder die zweite Inkubationskammer (20) eine zweite Verbindungsöffnung (13; 23) mit einer daran angeordneten Verbindungseinrichtung (14; 24) umfasst, und
wobei die Verbindungseinrichtung (32) der dritten Inkubationskammer (30) mit der zweiten Verbindungseinrichtung (14; 24) der ersten Inkubationskammer (10) oder mit der zweiten Verbindungseinrichtung (14; 24) der zweiten Inkubationskammer (20) verbunden ist.

## Geänderte Patentansprüche

### Geänderte Patentansprüche gemäss Regel 137(2) EPÜ.

1. Modulares Inkubationssystem (100), umfassend:
eine Inkubationsvorrichtung (1) umfassend:
eine erste Inkubationskammer (10); und
eine Schnittstelle (18) in einer Außenwand der ersten Inkubationskammer (10),
wobei in der ersten Inkubationskammer (10) angeordnet sind:
ein Probenträgerhalter (15), und
eine Untersuchungsvorrichtung (16) zur Untersuchung einer Probe, die in
dem Probenträgerhalter (15) angeordnet ist,
wobei die erste Inkubationskammer (10) eine Verbindungsöffnung (11) zum Verbinden mit einer zweiten Inkubationskammer (20) und eine Einlassöffnung (17) zum Einbringen der Probe in die erste Inkubationskammer (10) umfasst,
wobei an der Verbindungsöffnung (11) eine Verbindungseinrichtung (12) angeordnet ist, und
wobei die Verbindungseinrichtung (12) mit einer Verbindungseinrichtung (22), die an einer Verbindungsöffnung (21) der zweiten Inkubationskammer (20) angeordnet ist, verbindbar ist,
wobei die Schnittstelle (18) über einen Anschluss für eine gasführende und/oder flüssigkeitsführende Verbindung verfügt, und
eine zweite Inkubationskammer (20) mit einer Verbindungsöffnung (21),
wobei an der Verbindungsöffnung (21) der zweiten Inkubationskammer (20) eine Verbindungseinrichtung (22) angeordnet ist,
wobei die Verbindungseinrichtung (12) der ersten Inkubationskammer (10) mit der Verbindungseinrichtung (22) der zweiten Inkubationskammer (20) verbunden ist,
wobei in einer Außenwand der zweiten Inkubationskammer (20) eine Schnittstelle (28) für einen Anschluss einer gasführenden und/oder flüssigkeitsführenden Verbindung angeordnet ist, und
wobei die Schnittstelle (18) der ersten Inkubationskammer (10) mit der Schnittstelle (28) der zweiten Inkubationskammer (20) verbunden ist, sodass eine geschlossene gasführende und/oder flüssigkeitsführende Verbindung vorliegt.

2. Inkubationssystem (100) nach Anspruch 1, wobei die Verbindungseinrichtung (12) der ersten Inkubationskammer (10) eine Komponente einer Schraubverbindung, einer magnetischen Verbindung mit Positioniermechanik, eines Bajonettverschlusses und/oder einer Einhängeverbindung, beispielsweise mit einem Spannverschluss, umfasst.

3. Inkubationssystem (100) nach Anspruch 1, wobei die Schnittstelle (18) eine Steckverbindung, insbesondere mit einem Dichtungselement, umfasst.

4. Inkubationssystem (100) nach einem der vorangegangenen Ansprüche, wobei die erste Inkubationskammer (10) eine zweite Verbindungsöffnung (13) aufweist, an der eine zweite Verbindungseinrichtung (14) angeordnet ist.

5. Inkubationssystem (100) nach einem der vorangegangenen Ansprüche, weiterhin umfassend eine Proben-Versorgungseinrichtung (40) zur Bereitstellung eines Gases oder einer Flüssigkeit für die in dem Probenträgerhalter (15) angeordnete Probe,
wobei die Proben-Versorgungseinrichtung (40) insbesondere eine Pumpe mit einem Flüssigkeitsreservoir umfasst.

6. Inkubationssystem (100) nach einem der vorangegangenen Ansprüche, weiterhin umfassend ein Heizelement, das ausgebildet ist, eine Temperatur in der ersten Inkubationskammer (10) und/oder der zweiten Inkubationskammer (20) einzustellen, und/oder eine Kammer-Versorgungseinrichtung (41), die ausgebildet ist, eine Luftfeuchtigkeit und/oder Gaskonzentration, insbesondere CO₂- und/oder O₂-Konzentration, in der ersten Inkubationskammer (10) und/oder der zweiten Inkubationskammer (20) einzustellen.

7. Inkubationssystem (100) nach Anspruch 6, wobei das Heizelement ausgebildet ist, die Temperatur in der ersten Inkubationskammer (10) und in der zweiten Inkubationskammer (20) unabhängig voneinander einzustellen, und/oder
wobei die Kammer-Versorgungseinrichtung (41) ausgebildet sind, die Luftfeuchtigkeit und/oder die Gaskonzentration in der ersten Inkubationskammer (10) und in der zweiten Inkubationskammer (20) unabhängig voneinander einzustellen.

8. Inkubationssystem (100) nach einem der vorangegangenen Ansprüche, weiterhin umfassend einen Luftverteiler (43), beispielsweise einen Ventilator, für einen Austausch einer Temperatur, Gaskonzentration und/oder einer Luftfeuchtigkeit zwischen der ersten Inkubationskammer (10) und der zweiten Inkubationskammer (20).

9. Inkubationssystem (100) nach einem der vorangegangenen Ansprüche, wobei die erste Inkubationskammer (10) und/oder die zweite Inkubationskammer (20) gegenüber einer Umgebung thermisch isoliert sind/ist.

10. Inkubationssystem (100) nach einem der vorangegangenen Ansprüche, weiterhin umfassend:
einen Temperatursensor (42), Luftfeuchtesensor (42) und/oder Gaskonzentrationssensor (42) zur Bestimmung mindestens eines Parameters von Temperatur, Luftfeuchtigkeit und/oder Gaskonzentration in der ersten Inkubationskammer (10) und/oder der zweiten Inkubationskammer (20).

11. Inkubationssystem (100) nach Anspruch 10, weiterhin umfassend eine Regeleinrichtung (50), die derart ausgebildet ist, den besagten Parameter auf Grundlage eines zugehörigen Messwerts von dem Sensor (42) oder den Sensoren (42) zu regeln.

12. Inkubationssystem (100) nach einem der vorangegangenen Ansprüche, weiterhin umfassend eine dritte Inkubationskammer (30),
wobei die dritte Inkubationskammer (30) eine Verbindungsöffnung (31) mit einer daran angeordneten Verbindungseinrichtung (32) umfasst,
wobei die erste Inkubationskammer (10) oder die zweite Inkubationskammer (20) eine zweite Verbindungsöffnung (13; 23) mit einer daran angeordneten Verbindungseinrichtung (14; 24) umfasst, und
wobei die Verbindungseinrichtung (32) der dritten Inkubationskammer (30) mit der zweiten Verbindungseinrichtung (14; 24) der ersten Inkubationskammer (10) oder mit der zweiten Verbindungseinrichtung (14; 24) der zweiten Inkubationskammer (20) verbunden ist.
